# EUROPÄISCHE PATENTSCHRIFT

(11) **EP 4 064 978 B1**
(45) Veröffentlichungstag und Bekanntmachung des Hinweises auf die Patenterteilung: **15.11.2023**
(21) Anmeldenummer: 20817257.7
(22) Anmeldetag: 30.11.2020
(51) Int. Cl.: A61B 5/022, A61B 5/1455, A61B 5/00, A61B 5/0225, A61B 5/021

(54) **PHOTOPLETHYSMOGRAPHISCHE BLUTDRUCKMESSEINRICHTUNG MIT ABNEHMBARER FINGERMANSCHETTE**
PHOTOPLETHYSMOGRAPHIC BLOOD PRESSURE MEASURING DEVICE WITH REMOVABLE FINGER CUFF
DISPOSITIF DE MESURE DE LA PRESSION ARTÉRIELLE PHOTOPLÉTHYSMOGRAPHIQUE AVEC MANCHON POUR DOIGT AMOVIBLE

(30) Priorität: 01.12.2019 DE 202019004899 U
(43) Veröffentlichungstag der Anmeldung: 05.10.2022
(73) Patentinhaber: Pulsion Medical Systems SE, 85622 Feldkirchen (DE)
(72) Erfinder: WEBER, Aaron, 85570 Markt Schwaben (DE); HEIN, André, 73728 Esslingen am Neckar (DE); THALMEIER, Thomas, 84405 Dorfen (DE)
(74) Vertreter: Kehl, Ascherl, Liebhoff & Ettmayr Patentanwälte Partnerschaft mbB
(86) Internationale Anmeldenummer: PCT/EP2020/083963
(87) Internationale Veröffentlichungsnummer: WO 2021/110597

(56) Entgegenhaltungen:
- WO-A1-2017/143366
- WO-A2-2012/032413
- JP-A- H06 125 881

## Beschreibung

### Technisches Gebiet

Die vorliegende Erfindung betrifft eine Messvorrichtung zur kontinuierlichen Bestimmung des intra-arteriellen Blutdruckes an zumindest einem Finger einer Hand.

### Stand der Technik

Der (insbesondere arterielle) Blutdruck eines Patienten ist eine der wichtigsten Messgrößen in der Medizintechnik, und die bekannte zugehörige, insbesondere auch nichtinvasive Messtechnik überaus vielfältig. Dies gilt vor allem für Messtechnik zur kontinuierlichen Überwachung des Blutdrucks über einen längeren Zeitraum, beispielsweise in der Intensivmedizin aber auch in der Notfallmedizin und während operativen Eingriffen.

Aus Gründen der guten Zugänglichkeit ist die Blutdruckmesseinrichtung dabei oft an Gliedmaßen des Patienten angebracht, beispielsweise ein applanationstonometrischer Sensor bei der Radialarterie am Unterarm oder ein photoplethysmographisch nach der sogn. "Vascular Unloading Technique" nach Peňáz betriebener Fingersensor. Derartige Druckmesseinrichtungen sind beispielsweise aus US 4,406,289, US 4,524,777, US 4,726,382, WO 2010/050798 A1, WO 2000/059369 A1, WO 2011/045138 A1, WO 2011/051819 A1, WO 2011/051822 A1, WO 2012/032413 A1 und WO 2017/143366 A1 bekannt.

Bei der Vascular Unloading Technique wird nahinfrarotes Licht in einen Finger eingestrahlt, und anhand des mittels eines Photodetektors aufgefangenen nicht absorbierten Anteils der pulsatile (pulsförmige) Blutfluss (tatsächlich das sich verändernde Blutvolumen) im Finger bestimmt. Für dieses, auch Photoplethysmographie (PPG) genannte, Verfahren wird das (nahinfrarote) Licht üblicherweise mit Hilfe von einer oder mehreren Leuchtdioden (LED), die mit einer oder mehreren Wellenlängen arbeiten, erzeugt sowie mit Hilfe von einer oder mehreren lichtempfindlichen Empfängerdioden (Photodioden) detektiert. Anstelle von Dioden sind auch andere Arten von Photoempfängern grundsätzlich geeignet.

Ein Regelungssystem hält nun den plethysmographisch registrierten Fluss (bzw. das detektierte Blutvolument) und somit das resultierende photoplethysmographische Signal (Volumssignal v(t)) konstant, in dem ein Gegendruck in einer Manschette (Cuffpressure) pc(t) am Finger aufgebracht wird. Dieser Gegendruck pc(t) wird dabei üblicherweise von einem schnellen Ventil oder Ventilsystem im Zusammenspiel mit einer Pumpe geregelt. Die diesbezügliche Ansteuerung des Ventils bzw. des Ventilsystems wird von einer Regelungseinheit durchgeführt, die vorzugweise mit einem Microcomputer realisiert wird. Die wesentlichen Eingangssignale sind dabei das PPG-Signal v(t) und der Manschettendruck pc(t). Der für die Konstanthaltung des PPG-Signales v(t) notwendige Druck pc(t) entspricht nun dem intra-arteriellen Blutdruck pa(t).

Dafür ist es erforderlich, dass sich der Manschettendruck pc(t) mindestens so schnell verändern lässt, wie sich auch der intra-arterielle Blutdruck pa(t) ändert, damit die Echtzeitbedingung erfüllt ist. Die obere Grenzfrequenz von pa(t) und somit die höchste Druckänderungsgeschwindigkeit liegt oberhalb von zumindest 20Hz, was für ein Druckregelsystem durchaus eine Herausforderung darstellt. Hieraus folgt, dass sich die Druckregelung durch ein Ventil bzw. Ventilsystem vorteilhafterweise in unmittelbarer Nähe der Manschette befindet. Bei zu langen Luftleitungen droht der Verlust dieser Grenzfrequenzbedingung wegen der Tiefpasswirkung der Leitungen.

Aus der US 4,406,289 ist ein mechanisches Ventil bekannt, das den Gegendruck in der Fingermanschette mit der gewünschten Genauigkeit regelt, wenn es mit einer linear arbeitenden Pumpe versorgt wird. Das Ventil ist in einem Gehäuse am distalen Unterarm untergebracht und versorgt so die Fingermanschette über einen kurzen Schlauch mit dem Druck pc(t).

Die US 4,524,777 beschreibt ein Druckerzeugungssystem für die Vascular Unloading Technique, wobei ebenfalls ein konstanter Manschettendruck Pc mit einer linearen Pumpe erzeugt wird, der mit Druckschwankungen Δpc(t) aus einem parallel geschalteten "Shaker" bzw. einem " Driving Actuator" überlagert wird.

In US 4,726,382 ist eine Fingermanschette für die Vascular Unloading Technique offenbart, die Schlauchanschlüsse für die Versorgung mit dem Manschettendruck pc(t) besitzt. Die Länge der Luftschläuche reicht bis zum Druckerzeugungssystem, das wiederum am distalen Unterarm befestigt ist.

Die WO 2000/059369 A1 beschreibt ebenfalls ein Druckerzeugungssystem für die Vascular Unloading Technique. Das Ventilsystem besteht hier aus einem separaten Einlass- und einem separaten Auslassventil. Während bei den Patentschriften US 4,406,289 und US 4,524,777 eine relativ lineare Proportionalpumpe verwendet werden muss, erlaubt dieses System die Verwendung von einfachen kostengünstigen Pumpen, da störende Oberwellen durch die Anordnung der Ventile eliminiert werden können. Ferner kann der Energieverbrauch der einfachen Pumpe durch das Ventilprinzip wesentlich reduziert werden.

Aus der WO 2004/086963 A1 ist ein System für die Vascular Unloading Technique bekannt, bei dem in einem Finger kontinuierlich der Blutdruck bestimmt werden kann, während im benachbarten Finger eine Kontrolle der Messqualität vorgenommen wird ("Watch Dog"-Funktion). Nach einer Zeit wechselt das System automatisch den "Messfinger" mit dem "Überwachungsfinger" .

Die WO 2005/037097 A1 beschreibt ein Regelungssystem für die Vascular Unloading Technique mit mehreren ineinander verflochtene Regelkreisen.

Die WO 2010/050798 A1 offenbart ein am distalen Unterarm befestigtes Druckerzeugungssystem ("Frontend") mit nur einem Ventil, an dem eine Fingermanschette für die Vascular Unloading Technique angebracht werden kann.

Bei einem in WO 2011/045138 A1 beschriebenen Druckerzeugungssystem für die Vascular Unloading Technique wird - ähnlich wie aus der WO 2000/059369 bekannt - die Energieaufnahme der Pumpe reduziert, und es können Oberwellen eliminiert werden.

Die WO 2011/051819 A1 offenbart eine mittels digitaler Elektronik verbesserte Implementierung der Vascular Unloading Technique zur Erhöhung der Stabilität sowie zur weiteren Miniaturisierung.

In WO 2011/051822 A1 ist ein Verfahren für die Vascular Unloading Technique beschrieben, bei dem die gemessenen Signale v(t) und pc(t) zur Erhöhung langfristiger Stabilität und zum Ermitteln weiterer hämodynamischer Parameter verarbeitet werden. Insbesondere werden ein Verfahren zur Elimination von Effekten, die von vasomotorischer Veränderungen der Fingerarterien herrühren, sowie eine Methode zur Bestimmung des Herzzeitvolumens (*Cardiac Ouput* CO) offenbart.

Die WO 2012/032413 A1 beschreibt neuartige Fingersensoren, die ein Wegwerfteil (Disposable) zur einmaligen Verwendung besitzen. Dabei ist die mit dem Finger in Berührung kommende Manschette aus hygienischen Gründen im Wegwerfteil untergebracht, wohingegen das zugehörige Druckerzeugungs- und Druckregelsystem in einem wiederverwertbaren Teil untergebracht ist. Entsprechend ist hier eine trennbare pneumatische Verbindung zwischen Wegwerfteil und wiederverwertbarem Teil vorzusehen.

In der Regel wird das Druckerzeugungs- und Druckregelsystem im Stand der Technik am distalen Unterarm, proximal des Handgelenkes, angebracht, was wesentliche Nachteile mit sich bringt: Diese Stelle wird häufig für intravenöse Zugänge verwendet und auch der intraarterielle Zugang am distalen Ende der Radialerterie sollte für Notfälle frei sein. Derartige Zugänge können durch das Druckerzeugungs- und Druckregelsystem und dessen Befestigung blockiert werden. Außerdem kann im Betrieb das System verrutschen bzw. kippen. Dies kann sich nachteilig auf den Sitz der Sensoren auswirken. Der Sitz der Sensoren würde sich außerdem verbessern, wenn sich der zu messende Finger bzw. die entsprechende Hand in einer gewissen Ruhelage befindet.

Zur Überwindung dieser Problematik schlägt die Druckschrift WO 2017/143366 A1 ein Messsystem zur kontinuierlichen Bestimmung des intra-arteriellen Blutdruckes an zumindest einem Finger einer Hand, mit zumindest einem Fingersensor, mit einem plethysmographischen System, mit zumindest einer Lichtquelle, vorzugsweise LED, mit einer
oder mehreren Wellenlängen und zumindest einem Lichtaufnehmer und zumindest einer aufblasbaren Manschette, sowie mit einem Druckerzeugungssystem mit zumindest einem mit Hilfe des plethysmographischen Systems in Echtzeit geregelten Ventil zur Erzeugung eines Druckes in der Manschette, der im Wesentlichen dem intra-arteriellen Blutdruck im Finger entspricht, vor, wobei das Messsystem ein Gehäuse mit einer Oberfläche aufweist, die als Auflagefläche für den zumindest einen Finger und die angrenzenden Bereiche der Handinnenfläche dient. Die Hand ruht hier also auf einer Auflage, unter der sich wesentliche Komponenten befinden, die bei herkömmlichen Systemen am Unterarm befestigt waren.

Ähnlich der oben erwähnten WO 2012/032413 A1 ist die Manschette in einem vom Gehäuse (und somit von der Handauflage) trennbaren Wegwerfteil untergebracht. Entsprechend ist wiederum eine trennbare pneumatische Verbindung zwischen Wegwerfteil und wiederverwertbarem Teil vorzusehen.

Aus JP H06-125881 A ist ein Pulswellenmesssystem mit einer Fingermanschette bekannt, wobei eine optische Sensoreinheit außerhalb der Manschette an der Spitze des Fingers im Bereich des Nagels angebracht ist. Das System weist ein Gehäuse mit einer Oberfläche auf, die als Auflagefläche für den Finger und die angrenzenden Bereiche der Handinnenfläche dient. Eine Trennung in einen Wegwerfteil und wiederverwertbarem Teil ist hier nicht vorgesehen. Die Auflagefläche ist Teil des wiederverwertbaren Gesamtsystems.

Bei den bekannten Systemen können hygienische Probleme auftreten, da die Patientenhand leicht mit wiederverwendbaren Teilen der Vorrichtungen in Berührung kommen kann oder sogar bestimmungsgemäß stets mit wiederverwendbaren Teilen der entsprechenden Vorrichtung in Berührung kommt. Ferner können Probleme bei längerer Benutzung auftreten, da sich die Hand des Patienten verkrampfen oder Kanten der Vorrichtung in die Hand einschneiden können. Zu beachten ist dabei, dass Patienten in der Vorbereitung zu einer Operation, während einer Operation, insbesondere im sedierten Zustand, und bei der anschließenden Überwachung im Aufwachraum oft stundenlang an derartige Vorrichtungen angeschlossen sind. Dabei kann es bei lokalen Druckstellen zu Schädigungen des Patientengewebes kommen. Der Patient kann vor allem im sedierten Zustand die Lage seiner Hand und seines Arms nicht gewollt steuern, um so der Bildung von Druckstellen entgegenzuwirken.

### Darstellung der Erfindung

Angesichts der bei herkömmlichen Systemen bestehenden Einschränkungen ist es Aufgabe der vorliegenden Erfindung, Messvorrichtungen der eingangs genannten Art in Hinblick auf Hygiene und/oder Sicherheit und/oder Komfort für den Patienten zu verbessern.

Gemäß einem Aspekt der vorliegenden Erfindung wird diese Aufgabe mit einem Manschetttenteil gemäß Anspruch 1 gelöst. Bevorzugte Ausführungsformen der Erfindung können gemäß einem der abhängigen Ansprüche umgesetzt werden. Die vorliegende Erfindung stellt somit insbesondere eine Messvorrichtung zur kontinuierlichen Bestimmung des intra-arteriellen Blutdruckes an zumindest einem Finger einer Hand bereit, welche einen Basisteil, einen mit dem Basisteil werkzeugfrei verbindbaren und vom Basisteil werkzeugfrei trennbaren Manschettenteil, der mindestens eine ringartige Aufnahmeröhre zum Aufnehmen eines Abschnitts eines durch die Aufnahmeröhre hindurchgeführten Fingers einer Hand und mindestens ein in der Aufnahmeröhre angeordnetes, mit einem Fluid füllbares Manschettenpolster aufweist, eine Strahlungsquelle zum Aussenden von Licht in den Finger durch eine optische Emissionsfläche, einen Photodetektor zum Detektieren eines durch eine optische Kollektorfläche aufgefangenen, in dem Finger nicht absorbierten Anteils des Lichts, und ein zumindest teilweise im Basisteil angeordnetes Druckregelsystem zum Regeln eines Fluiddrucks in dem Manschettenpolster in Abhängigkeit des detektierten nicht absorbierten Anteils des Lichts aufweist, wobei der Manschettenteil ferner eine Handtellerauflage zum Auflegen des Handtellers der Hand und mindestens eine Fingerauflage, welche jeweils jeder der mindestens einen Aufnahmeröhre zugeordnet ist, zum Auflegen des über die jeweilige Aufnahmeröhre hinausragenden Teil des Fingers aufweist, und der Manschettenteil den Basisteil in Längsrichtung nach vorne und nach hinten überragt, wobei die Längsrichtung als Richtung des bestimmungsgemäßen Einführens des Fingers in die Aufnahmeröhre bzw. der Finger in die Aufnahmeröhren definiert ist, die Handtellerauflage hinter und die mindestens eine Fingerauflage vor der Aufnahmeröhre angeordnet ist. Die Längsrichtung entspricht somit der axialen Richtung der Aufnahmeröhre(n).

Die Handtellerauflage und die Fingerauflage(n) sorgen für eine möglichst ermüdungsfreie und natürliche Haltung der Hand des Patienten während der Messung. Da der Manschettenteil den Basisteil in Längsrichtung nach vorne und nach hinten überragt, werden unerwünschte Berührungen des Patienten mit dem Basisteil vermieden, was der Betriebssicherheit der Messvorrichtung, der Hygiene, Patientensicherheit und dem Patientenkomfort dienlich ist.

Gemäß einer vorteilhaften Ausführungsform überragt der Basisteil den Manschettenteil auch nicht in Querrichtung zumindest über einen Großteil der Länge des Basisteils. So können unerwünschte Berührungen des Patienten mit dem Basisteil noch besser vermieden werden.

Gemäß eine vorteilhaften Weiterbildung überdeckt der Manschettenteil in zumindest einer, insbesondere einer zu Längs- und Querrichtung jeweils parallelen, Projektionsebene den Basisteil vollständig, wenn eine ggf. vom Basisteil ausgehende Kabelverbindung nicht dem Basisteil zugerechnet wird. Dies hat eine weiter erhöhte Sicherheit gegen Berührungen des Basisteils durch den Patienten zur Folge.

Vorzugsweise weist die Handtellerauflage auf der bestimmungsgemäß der Hand zugewandten Seite eine konvexe Krümmung in Längsrichtung auf. Durch eine derartige Krümmung kann die Ergonomie deutlich verbessert werden. Zumindest abschnittsweise kann vorteilhafterweise auch eine konvexe Krümmung der Handtellerauflage in Querrichtung vorgesehen werden (auf der bestimmungsgemäß der Hand zugewandten Seite), welche die Ergonomie weiter verbessert. In einem hinteren Endbereich der Handtellerauflage kann auch eine konkave Krümmung (auf der bestimmungsgemäß der Hand zugewandten Seite) für den Handballen die Ergnomie vorteilhaft weiter verbessern.

Vorzugsweise weist die mindestens eine Fingerauflage auf der bestimmungsgemäß der Hand zugewandten Seite jeweils eine konkave Krümmung in Querrichtung auf. Dies gewährleistet, dass der jeweilige Finger ohne Verkrampfung längere Zeit weitgehend bewegungsfrei auf der Fingerauflage liegen kann.

Gemäß einer vorteilhaften Weiterbildung sind die Gesamtheit der mindestens einen Aufnahmeröhre(n), die Gesamtheit der mindestens einen Fingerablage, und die Handtellerauflage auf ihrer bestimmungsgemäß der Hand zugewandten Seite jeweils bezüglich einer gemeinsamen Längsachse symmetrisch ausgebildet. Hierdurch kann die Messvorrichtung mit ein und demselben Manschettenteil gleichermaßen für Links- und Rechtshänder verwendet werden.

Gemäß einer besonders bevorzugten Ausführungsform weist der Basisteil einen Kabelanschluss auf der so ausgerichtet ist, dass die axiale Richtung eines angeschlossenes Kabel in seiner Mittellage relativ zur Längsrichtung der Messvorrichtung in einem Winkel zwischen 5 und 45 Grad, vorzugsweise zwischen 10 und 30 Grad, schräg nach hinten zeigt, wenn der Winkel in einer waagrechten Winkelebene liegt oder auf eine waagrechte Winkelebene projiziert wird. Gleichzeitig oder stattdessen kann Kabelanschluss auch vorteilhafterweise bezüglich der Breite des Basisteils außermittig angeordnet sein.

Als Mittellage wird die Lage des Kabels verstanden, die der Anschluss vorgibt, wenn das angeschlossene Kabel geradlinig und ohne Auslenkung verläuft.

Eine entsprechende Kabelanbringung reduziert unerwünschte Kontakte mit dem Kabel. Außerdem lassen sich bei der Kabelführung unerwünschte Schlaufen und Knicke besser vermeiden: Die schräge bzw. seitlich versetzte, bei bestimmungsgemäß aufgelegter Hand in Richtung Unterarm weisende Anbringung des Kabels hat den Vorteil, dass das Kabel entlang des Patientenarms geführt werden kann, der Bereich des Handgelenks mit Sehnen und Gefäßen aber nicht am Kabel scheuert, und insbesondere auch der Karpaltunnel geschont wird.

Vorzugsweise ist dabei der Kabelanschluss in dem Bereich des Basisteils angeordnet, der in einer zu Längs- und Querrichtung senkrechten Richtung von der Handtellerauflage überdeckt wird.

Gemäß einer bevorzugten Ausführungsform sind alle Kanten der Aufnahmeröhre(n) abgerundet und/oder gefast. So lassen sich Druck- und Scheuerstellen an den Fingern sowie das unerwünschte Abschnüren von Blutgefäßen vermeiden.

Ebenfalls bevorzugt können alle Außenkanten des Aufnahmekörpers abgerundet und/oder gefast sein, um Druck- und Scheuerstellen auf der Patientenhaut vorzubeugen.

Gemäß einem weiteren Aspekt der vorliegenden Erfindung wird ein System bereitgestellt, das eine Messvorrichtung gemäß einer der obigen Ausführungsformen aufweist und dazu mindestens einen weiteren gegen den Manschettenteil werkzeugfrei austauschbaren Manschettenteil, der mindestens eine ringartige Aufnahmeröhre zum Aufnehmen eines Abschnitts eines durch die Aufnahmeröhre hindurchgeführten Fingers einer Hand und mindestens ein in der Aufnahmeröhre angeordnetes, mit einem Fluid füllbares Manschettenpolster, eine Handtellerauflage zum Auflegen des Handtellers der Hand und mindestens eine Fingerauflage, welche jeweils jeder der mindestens einen Aufnahmeröhre zugeordnet ist, zum Auflegen des über die jeweilige Aufnahmeröhre hinausragenden Teil des Fingers aufweist, wobei die Aufnahmeröhre und/oder die mindestens eine Fingerauflage und/oder die Handtellerauflage des Manschettenteils und des weiteren Manschettenteils jeweils unterschiedlich voneinander dimensioniert sind. So können durch Wechsel der Manschettenteile für unterschiedlich große Patientenhände ergonomisch geeignete Konfigurationen der Messvorrichtung geschaffen werden.

Grundsätzlich kann jede im Rahmen der vorliegenden Anmeldung beschriebene bzw. angedeutete Variante der Erfindung besonders vorteilhaft sein, je nach wirtschaftlichen, technischen und ggf. medizinischen Bedingungen im Einzelfall. Soweit nichts gegenteiliges dargelegt ist, bzw. soweit grundsätzlich technisch realisierbar, sind einzelne Merkmale der beschriebenen Ausführungsformen austauschbar oder miteinander sowie mit per se aus dem Stand der Technik bekannten Merkmalen kombinierbar.

Die Erfindung wird nachfolgend beispielhaft anhand der beigefügten schematischen Zeichnungen näher erläutert. Die Zeichnungen sind nicht maßstabsgetreu; insbesondere entsprechen Verhältnisse der einzelnen Abmessungen zueinander aus Gründen der Anschaulichkeit nicht unbedingt den Abmessungsverhältnissen in tatsächlichen technischen Umsetzungen. Einander entsprechende Elemente sind in den einzelnen Figuren mit denselben Bezugszeichen gekennzeichnet.

### Kurze Beschreibung der Figuren

- Figur 1: zeigt eine erfindungsgemäße Messvorrichtung in einer Seitenansicht, wobei ein eine ergonomische Handauflage und einen Finger-Aufnahmeteil aufweisender Manschettenteil auf einen ein Druckregelsystem enthaltenden Basisteil aufgesetzt ist,
- Figur 2: zeigt eine perspektivische Ansicht der Messvorrichtung aus Figur 1 von schräg oben,
- Figur 3: zeigt eine perspektivische Ansicht der Messvorrichtung aus Figur 1 von schräg unten,
- Figur 4: zeigt einen den Manschettenteil wie den in Figur 3 gezeigten von schräg unten, jedoch ohne zugehörigen Basisteil, wobei die Manschettenpolster nicht dargestellt sind,
- Figur 5: zeigt eine Ansicht auf die Messvorrichtung aus Fig. 1 von hinten, d.h. von links in Fig. 1,
- Figur 6: zeigt die Messvorrichtung aus Fig. 1 in der Draufsicht.

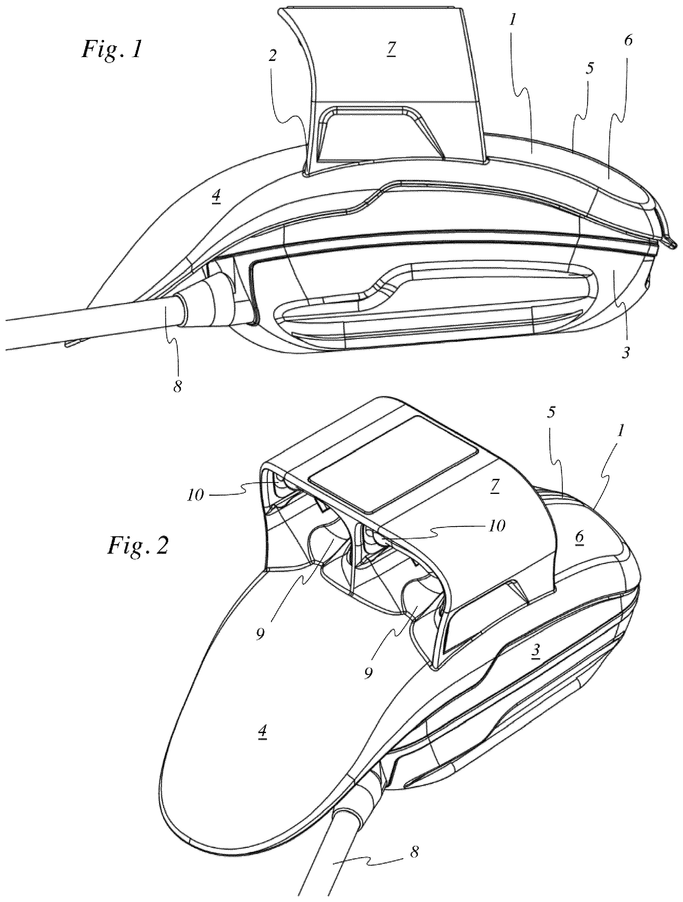

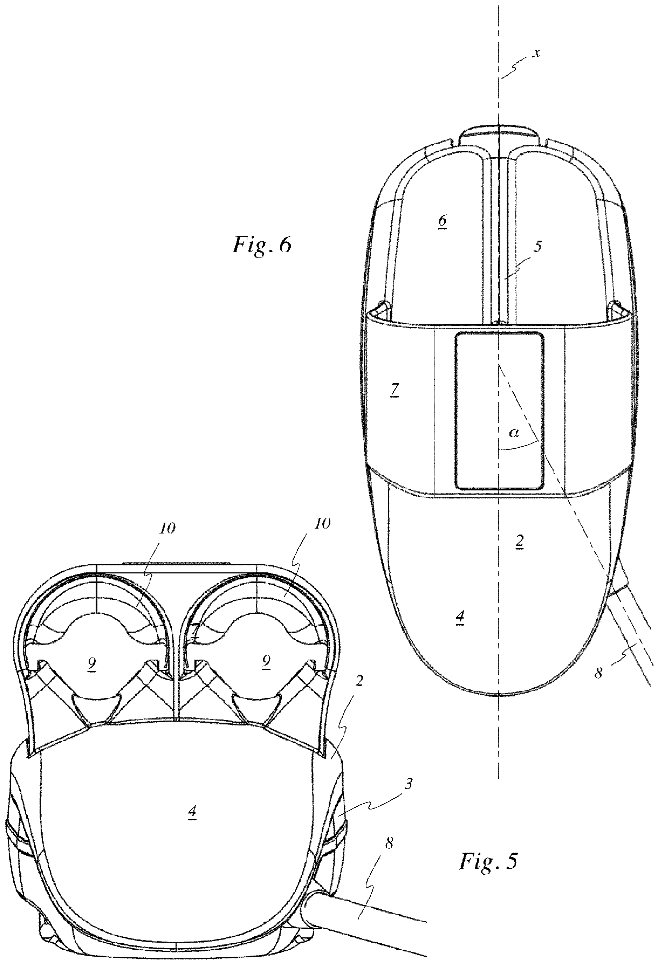

Die Blutdruckmesseinrichtung 1 ist als photoplethysmographisches Messsystem ausgeführt, das entsprechend der sogenannten "Vascular Unloading Technique" funktioniert. Messtechnische Komponenten, d.h. insbesondere optische und elektronische Bauteile sowie mechanische Bauteile des im Basisteil 3 untergebrachten Druckerzeugungs- und Druckregelsystems können dabei grundsätzlich ähnlich wie im eingangs genannten Stand der Technik implementiert werden. Der auf den Basisteil 3 aufgesetzte Manschettenteil weist eine ergonomische Handtellerauflage 4, zwei durch einen Steg 5 voneinander abgeteilte ergonomische Fingerauflagen 6, sowie einen Aufnahmeteil 7 auf, welcher zwei Aufnahmeröhren 9 zur Aufnahme zweier Finger umfasst, wie in Fig. 2 und Fig. 5 erkennbar. Die Fingerauflagen 6 besitzen eine konkave Krümmung in ihrer Querrichtung, so dass die Finger jeweils bequem darauf ruhen können. Die Kanten an den Rändern der Aufnahmeröhren 9 sind gefast.

Nach obiger Definition entspricht die linke Seite der Fig. 1 der Richtungsangabe "hinten", die rechte Seite der Fig. 1 der Richtungsangabe "vorne". Die obere Seite der Fig. 6 entspricht demgemäß der Richtungsangabe "hinten", dir untere Seite der Fig. 6 der Richtungsangabe "hinten".

Wie in Fig. 1 erkennbar, weist die Handtellerauflage 4 in Längsrichtung oben (d.h. auf der der bestimmungsgemäß der Patientenhand zugewandten Seite) eine konvexe Krümmung auf, um die Ergonomie zu verbessern. In diesem Bereich ist die Handtellerauflage auch in Querrichtung konvex gekrümmt. In einem Endbereich (links in Fig. 1) ist für den Handballen ein konkav in Längsrichtung gekrümmter Bereich vorgesehen.

Der Manschettenteil 2 überragt den Basisteil 3 sowohl nach vorne als auch nach hinten. Wie aus Fig. 6 ersichtlich, überdeckt der Manschettenteil 2 den Basisteil 3 fast vollständig, d.h. zu mehr als 90%.

Der Aufnahmeteil 7 ist, wie in der perspektivischen Ansicht von Fig. 2 (Blickrichtung von schräg rechts oben in Fig. 1) zur Aufnahme von zwei Fingern gestaltet, was wechselweises Messen an beiden Fingern möglich macht. Aus hygienischen Gründen ist der Manschettenteil 2 zusammen mit der Handtellerauflage 4 und Fingerauflage 6 als Einwegartikel ausgeführt, der mittels einer Steckverbindung werkzeugfrei lösbar am wiederverwendbaren Basisteil 3 angebracht ist.

Über das schräg seitlich angebrachte, bei bestimmungsgemäß aufgelegter Hand in Richtung Unterarm weisende Kabel 8 wird das Druckregelsystem im Basisteil 3 mit Druckluft versorgt, ferner dient das Kabel 8 zur Energiezufuhr für Druckregelsystem (nicht dargestellt), Lichtquellen (nicht dargestellt) und Photodetektoren (nicht dargestellt) bzw. zugehörige Steuerungs-, Verstärker- und Auswertungsschaltkreise in der Messvorrichtung 1. Messdaten können über eine geeignete elektronische Schnittstelle durch das Kabel 8 an einen Patientenmonitor ausgegeben werden.

Die seitliche, bei bestimmungsgemäß aufgelegter Hand in Richtung Unterarm weisende Anbringung des Kabels 8 hat den Vorteil, dass das Kabel entlang des Patientenarms geführt werden kann, der Bereich des Handgelenks mit Sehnen und Gefäßen aber nicht am Kabel 8 scheuert, und insbesondere auch der Karpaltunnel geschont wird.

Wie in Fig. 6 erkennbar, bildet die strichpunktiert angedeutete Mittelachse des nicht ausgelenkten Kabels 8 mit der der Längsrichtung entsprechenden Längsachse x des Manschettenteils einen Winkel α von hier 17 Grad und somit zwischen 10 und 30 Grad.

Die Gesamtheit der Aufnahmeröhren 9, d.h. der Aufnahmeteil 7, sowie die Fingerauflagen 6 und die Handtellerauflage 9 sind bezüglich der Längsachse x des Manschettenteils symmetrisch ausgebildet. Somit ist der Manschettenteil für die rechte und linke Hand gleichermaßen geeignet.

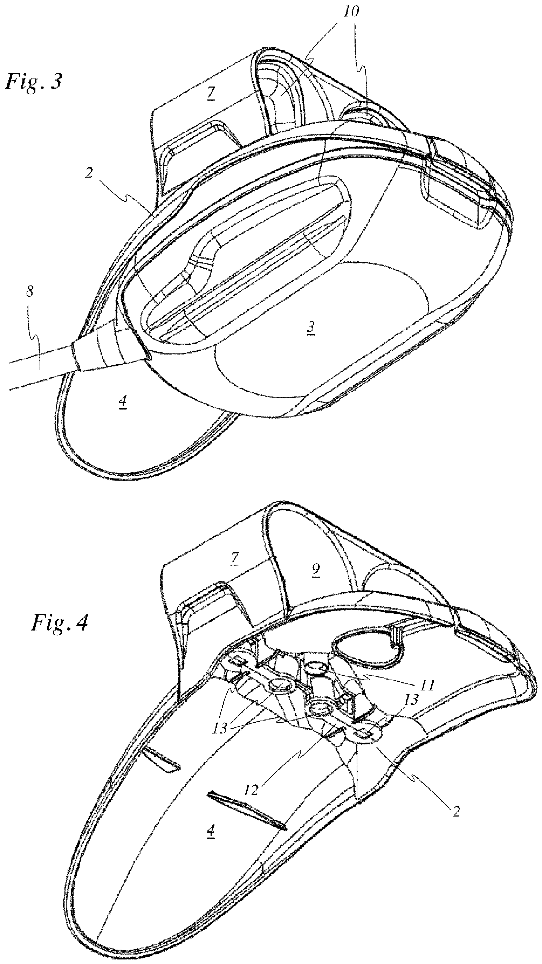

Die beiden in den Aufnahmeröhren 9 angeordnete aufblasbaren Manschettenpolster 10 sind jeweils über einen Anschluss 11 an der Schnittstelle zwischen Manschettenteil 2 und Basisteil 3 mit dem Druckerzeugungs- und Druckregelsystem verbunden. In der Darstellung des Manschettenteils 2 in Fig. 4 ist einer der beiden Anschlüsse 11 durch die Traverse 12 verdeckt, welche Lichtleiter 13 trägt, über die Licht von den im Basisteil 3 angeordneten Lichtquellen zu den Fingern bzw. von den Fingern zu den im Basisteil 3 angeordneten Photosensoren geleitet wird. Am Anschluss 11 befindet sich dabei vorzugsweise eine Ventilvorrichtung, so dass der Anschluss 11 basisteilseitig bündig mit dem Gehäuse des Basisteils 3 abschließt, wenn Basisteil 3 und Manschettenteil 2 nicht miteinander verbunden sind.

## Patentansprüche

1. Messvorrichtung (1) zur kontinuierlichen Bestimmung des intra-arteriellen Blutdruckes an zumindest einem Finger einer Hand, welche folgendes aufweist:
einen Basisteil (3),
einen mit dem Basisteil (3) werkzeugfrei verbindbaren und vom Basisteil (3) werkzeugfrei trennbaren Manschettenteil (2), der mindestens eine ringartige Aufnahmeröhre (9) zum Aufnehmen eines Abschnitts eines durch die Aufnahmeröhre (9) hindurchgeführten Fingers einer Hand und mindestens ein in der Aufnahmeröhre (9) angeordnetes, mit einem Fluid füllbares Manschettenpolster aufweist,
eine Strahlungsquelle zum Aussenden von Licht in den Finger durch eine optische Emissionsfläche,
einen Photodetektor zum Detektieren eines durch eine optische Kollektorfläche aufgefangenen, in dem Finger nicht absorbierten Anteils des Lichts, und
ein zumindest teilweise im Basisteil (3) angeordnetes Druckregelsystem zum Regeln eines Fluiddrucks in dem Manschettenpolster in Abhängigkeit des detektierten nicht absorbierten Anteils des Lichts,
**dadurch gekennzeichnet,**
**dass** der Manschettenteil (2) ferner eine Handtellerauflage (4) zum Auflegen des Handtellers der Hand und mindestens eine Fingerauflage (6), welche jeweils jeder der mindestens einen Aufnahmeröhre (9) zugeordnet ist, zum Auflegen des über die jeweilige Aufnahmeröhre (9) hinausragenden Teil des Fingers aufweist,
und der Manschettenteil (2) den Basisteil (3) in Längsrichtung nach vorne und nach hinten überragt, wobei die Längsrichtung als Richtung des bestimmungsgemäßen Einführens des Fingers in die Aufnahmeröhre (9) bzw. der Finger in die Aufnahmeröhren (9) definiert ist, die Handtellerauflage (4) hinter und die mindestens eine Fingerauflage (6) vor der Aufnahmeröhre (9) angeordnet ist.

2. Messvorrichtung (1) gemäß Anspruch 1, wobei der Basisteil (3) den Manschettenteil (2) zumindest über einen Großteil der Länge des Basisteils (3) in Querrichtung nicht überragt.

3. Messvorrichtung (1) gemäß Anspruch 2, wobei der Manschettenteil (2) in zumindest einer, insbesondere einer zu Längs- und Querrichtung jeweils parallelen, Projektionsebene den Basisteil (3) vollständig überdeckt, wenn eine ggf. vom Basisteil (3) ausgehende Kabelverbindung nicht dem Basisteil (3) zugerechnet wird.

4. Messvorrichtung (1) gemäß einem der vorangehenden Ansprüche, wobei die Handtellerauflage (4) auf der bestimmungsgemäß der Hand zugewandten Seite eine konvexe Krümmung in Längsrichtung aufweist.

5. Messvorrichtung (1) gemäß einem der vorangehenden Ansprüche, wobei die mindestens eine Fingerauflage (6) auf der bestimmungsgemäß der Hand zugewandten Seite jeweils eine konkave Krümmung in Querrichtung aufweist.

6. Messvorrichtung (1) gemäß einem der vorangehenden Ansprüche, wobei
a) die Gesamtheit der mindestens einen Aufnahmeröhre(n) (9),
b) die Gesamtheit der mindestens einen Fingerablage (6), und
c) die Handtellerauflage (4)
auf ihrer bestimmungsgemäß der Hand zugewandten Seite jeweils bezüglich einer gemeinsamen Längsachse symmetrisch sind.

7. Messvorrichtung (1) gemäß einem der vorangehenden Ansprüche, wobei
der Basisteil (3) einen Kabelanschluss aufweist,
der so ausgerichtet ist, dass die axiale Richtung eines angeschlossenen Kabels (8) in seiner Mittellage, d.h. ohne Auslenkung des Kabels, relativ zur Längsrichtung der Messvorrichtung (1) in einem Winkel zwischen 5 und 45 Grad schräg nach hinten zeigt, wenn der Winkel in einer waagrechten Winkelebene liegt oder auf eine waagrechte Winkelebene projiziert wird,
und/oder der bezüglich der Breite des Basisteils (3) außermittig angeordnet ist.

8. Messvorrichtung (1) gemäß Anspruch 7, wobei der Winkel zwischen 10 und 30 Grad beträgt.

9. Messvorrichtung (1) gemäß Anspruch 7 oder Anspruch 8, wobei der Kabelanschluss in dem Bereich des Basisteils (3) angeordnet ist, der in einer zu Längs- und Querrichtung senkrechten Richtung von der Handtellerauflage (4) überdeckt wird.

10. Messvorrichtung (1) gemäß einem der vorangehenden Ansprüche, wobei alle Kanten der Aufnahmeröhre(n) (9) abgerundet und/oder gefast sind.

11. Messvorrichtung (1) gemäß einem der vorangehenden Ansprüche, wobei alle Außenkanten der Aufnahmeröhre(n) (9) abgerundet und/oder gefast sind.

12. System aufweisend
eine Messvorrichtung (1) gemäß einem der Ansprüche 1-11, und
mindestens einen weiteren gegen den Manschettenteil (2) werkzeugfrei austauschbaren Manschettenteil (2), der mindestens eine ringartige Aufnahmeröhre (9) zum Aufnehmen eines Abschnitts eines durch die Aufnahmeröhre (9) hindurchgeführten Fingers einer Hand und mindestens ein in der Aufnahmeröhre (9) angeordnetes, mit einem Fluid füllbares Manschettenpolster, eine Handtellerauflage (4) zum Auflegen des Handtellers der Hand und mindestens eine Fingerauflage (6), welche jeweils jeder der mindestens einen Aufnahmeröhre (9) zugeordnet ist, zum Auflegen des über die jeweilige Aufnahmeröhre (9) hinausragenden Teil des Fingers aufweist,
wobei die Aufnahmeröhre (9) und/oder die mindestens eine Fingerauflage (6) und/oder die Handtellerauflage (4) des Manschettenteils (2) und des weiteren Manschettenteils (6) jeweils unterschiedlich voneinander dimensioniert sind.

## Claims

1. A measuring device (1) for continuous determination of intra-arterial blood pressure on at least one finger of a hand, which comprises:
a base part (3),
a cuff part (2) which is connectable to the base part (3) without tools and separatable from the base part (3) without tools and which comprises at least one ring-like receiving tube (9) for receiving a portion of a finger of a hand passed through the receiving tube (9), and at least one cuff pad which is arranged in the receiving tube and fillable with a fluid,
a radiation source for emitting light into the finger through an optical emission surface,
a photodetector for detecting a portion of the light collected by an optical collector surface and not absorbed in the finger, and
a pressure control system disposed at least partially in the base part (3) for controlling a fluid pressure in the cuff pad in response to the detected non-absorbed portion of the light,
**characterized in**
**that** the cuff part (2) further comprises a palm rest (4) for supporting the palm of the hand and at least one finger rest (6) associated with each of the at least one receiving tube (9), respectively, for supporting the portion of the finger protruding beyond the respective receiving tube (9),
and the cuff part (2) projects beyond the base part (3) in the longitudinal direction towards the front and towards the rear, the longitudinal direction being defined as the direction of intended insertion of the finger into the receiving tube (9) or of the fingers into the receiving tubes (9), the palm rest (4) being arranged behind and the at least one finger rest (6) being arranged in front of the receiving tube (9).

2. The measuring device (1) according to claim 1, wherein the base part (3) does not project beyond the cuff part (2) in the transverse direction over at least the major part of the length of the base part.

3. The measuring device (1) according to claim 2, wherein the cuff part (2) completely covers the base part (3) in at least one projection plane, in particular a projection plane parallel to both the longitudinal and transverse directions, if a cable connection possibly extending from the base part (3) is not considered part of the base part (3).

4. The measuring device according to any one of the preceding claims, wherein the palm rest comprises a convex curvature in longitudinal direction on the side intended to face the hand.

5. The measuring device (1) according to any one of the preceding claims, wherein the at least one finger rest (6) comprises a respective concave curvature in the transverse direction on the side intended to face the hand.

6. The measuring device (1) according to any one of the preceding claims, wherein
a) the entirety of the at least one receiving tube(s) (9),
b) the entirety of the at least one finger rest (6), and
c) the palm rest (4)
are, on their side intended to face the hand, each symmetrical with respect to a common longitudinal axis.

7. The measuring device (1) according to any one of the preceding claims, wherein
the base part (3) comprises a cable connection
which is oriented in such a way that the axial direction of a connected cable (8) in its central position, i.e. without deflection of the cable, points obliquely backwards relative to the longitudinal direction of the measuring device (1) at an angle of between 5 and 45 degrees when the angle lies in a horizontal angle plane or is projected onto a horizontal angle plane,
and/or which is off-center with respect to the width of the base part (3).

8. The measuring device according to claim 7, wherein the angle is between 10 and 30 degrees.

9. The measuring device (1) according to claim 7 or claim 8, wherein the cable connection is arranged in the region of the base part (3) which is covered by the palm rest (4) in a direction perpendicular to the longitudinal and transverse directions.

10. The measuring device (1) according to one of the preceding claims, wherein all edges of the receiving tube(s) (9) are rounded and/or chamfered.

11. The measuring device (1) according to any one of the preceding claims, wherein all outer edges of the receiving tube(s) (9) are rounded and/or chamfered.

12. A system comprising
a measuring device (1) according to any one of claims 1-11, and
at least one further cuff part (2) interchangeable with the cuff part (2) without tools, comprising at least one ring-like receiving tube (9) for receiving a portion of a finger of a hand passed through the receiving tube (9), and at least one fluid-fillable cuff pad arranged in the receiving tube (9), a palm rest (4) for supporting the palm of the hand, and at least one finger rest (6) associated with each of the at least one receiving tube (9) for supporting the portion of the finger extending beyond the respective receiving tube,
wherein the receiving tube (9) and/or the at least one finger rest (6) and/or the palm rest (4) of the cuff part (2) and of the further cuff part (6) are dimensioned differently from one another, respectively.

## Revendications

1. Dispositif de mesure (1) pour la détermination en continu de la pression sanguine intra-artérielle au niveau d'au moins un doigt d'une main, qui comprend ce qui suit :
une partie de base (3),
une partie de manchette (2) pouvant être reliée sans outil avec la partie de base (3) et pouvant être séparée sans outil de la partie de base (3), qui comprend au moins un tube de logement annulaire (9) pour le logement d'une portion d'un doigt d'une main, passé à travers le tube de logement (9), et au moins une garniture de manchette disposée dans le tube de logement (9) et pouvant être remplie d'un fluide,
une source de rayonnement pour l'émission de lumière dans le doigt à travers une surface d'émission optique,
un photodétecteur pour la détection d'une partie de la lumière capturée par une surface de collecteur optique, non absorbée par le doigt et
un système de régulation de pression disposé au moins partiellement dans la partie de base (3), pour la régulation d'une pression de fluide dans la garniture de manchette en fonction de la partie de lumière non absorbée détectée,
**caractérisé en ce que**
la partie de manchette (2) comprend en outre un appui de paume de main (4) pour l'appui de la paume de la main et au moins un appui de doigt (6) qui correspond respectivement à chacun des au moins un tube de logement (9), pour l'appui de la partie du doigt qui dépasse du tube de logement (9) respectif,
et la partie de manchette (2) dépasse de la partie de base (3) dans la direction longitudinale vers l'avant et vers l'arrière, dans lequel la direction longitudinale est définie comme la direction de l'introduction correcte du doigt dans le tube de logement (9) ou des doigts dans les tubes de logement (9), l'appui de paume de main (4) est disposé derrière et l'au moins un appui de doigt (6) est disposé devant le tube de logement (9).

2. Dispositif de mesure (1) selon la revendication 1, dans lequel la partie de base (3) ne dépasse pas dans la direction transversale de la partie de manchette (2) au moins sur une grande partie de la longueur de la partie de base (3).

3. Dispositif de mesure (1) selon la revendication 2, dans lequel la partie de manchette (2) recouvre entièrement la partie de base (3) dans au moins un plan de projection, plus particulièrement un plan de projection parallèle respectivement à la direction longitudinale et à la direction transversale, lorsqu'une liaison par câble, le cas échéant sortant de la partie de base (3), appartient à la partie de base (3).

4. Dispositif de mesure (1) selon l'une des revendications précédentes, dans lequel l'appui de paume de main (4) présente, sur le côté correctement orienté vers la main, une courbure convexe dans la direction longitudinale.

5. Dispositif de mesure (1) selon l'une des revendications précédentes, dans lequel l'au moins un appui de doigt (6) présente, sur le côté correctement orienté vers la main, une courbure concave dans la direction transversale.

6. Dispositif de mesure (1) selon l'une des revendications précédentes, dans lequel
a) l'ensemble des au moins un tube de logement (9),
b) l'ensemble de l'au moins un appui de doigt (6) et
c) l'appui de paume de main (4)
sont symétriques, sur leur côté correctement orienté vers la main, par rapport à un axe longitudinal commune.

7. Dispositif de mesure (1) selon l'une des revendications précédentes, dans lequel
la partie de base (3) comprend un raccordement par câble,
qui est conçu de sorte que la direction axiale d'un câble raccordé (8) est orientée, dans la position centrale, c'est-à-dire sans déviation du câble, par rapport à la direction longitudinale du dispositif de mesure (1), avec un angle entre 5 et 45 degrés vers l'arrière, lorsque l'angle se trouve dans un plan angulaire horizontal ou est projeté sur un plan angulaire horizontal,
et/ou qui est disposé de manière excentrée par rapport à la largeur de la partie de base (3).

8. Dispositif de mesure (1) selon la revendication 7, dans lequel l'angle est entre 10 et 30 degrés.

9. Dispositif de mesure (1) selon la revendication 7 ou la revendication 8, dans lequel le raccordement par câble est disposé dans la zone de la partie de base (3) qui est recouverte par l'appui de paume de main (4) dans une direction perpendiculaire à la direction longitudinale et à la direction transversale.

10. Dispositif de mesure (1) selon l'une des revendications précédentes, dans lequel toutes les arêtes du ou des tubes de logement (9) sont arrondies et/ou chanfreinées.

11. Dispositif de mesure (1) selon l'une des revendications précédentes, dans lequel toutes les arêtes extérieures du ou des tubes de logement (9) sont arrondies et/ou chanfreinées.

12. Système comprenant
un dispositif de mesure (1) selon l'une des revendications 1 - 11 et
au moins une autre partie de manchette (2) pouvant être remplacée sans outil par la partie de manchette (2), qui comprend au moins un tube de logement annulaire (9) pour le logement d'une portion d'un doigt d'une main, passé à travers le tube de logement (9) et au moins une garniture de manchette disposée dans le tube de logement (9) et pouvant être remplie d'un fluide, un appui de paume de main (4) pour l'appui de la paume de la main et au moins un appui de doigt (6) qui correspond respectivement à chacun des au moins un tube de logement (9), pour l'appui de la partie du doigt qui dépasse du tube de logement (9) respectif,
dans lequel le tube de logement (9) et/ou l'au moins un appui de doigt (6) et/ou l'appui de paume de main (4) de la partie de manchette (2) et de l'autre partie de manchette (6) sont dimensionnés de manière différente entre eux.
